Europäisches Patentamt

European Patent Office

Office européen des brevets

(11) Publication number: **0 028 793**
**B1**

# EUROPEAN PATENT SPECIFICATION

(45) Date of publication of patent specification: **02.11.83**

(21) Application number: **80106752.1**

(22) Date of filing: **03.11.80**

(51) Int. Cl.³: **C 12 M 1/34, C 12 Q 1/00**

(54) Method for measuring metabolic activity of animal or plant tissues.

(30) Priority: **02.11.79 JP 14268979**

(43) Date of publication of application:
**20.05.81 Bulletin 81/20**

(45) Publication of the grant of the patent:
**02.11.83 Bulletin 83/44**

(84) Designated Contracting States:
**CH DE FR GB LI**

(56) References cited:
**AU - A - 20 608**
**FR - A - 1 221 251**
**FR - A - 2 372 229**
**GB - A - 283 650**
**GB - A - 2 029 026**
**GB - B - 1 433 887**
**US - A - 3 403 081**
**US - A - 3 502 559**
**US - A - 4 065 357**
**US - A - 4 149 938**
**US - A - 4 160 205**

(73) Proprietor: KYOWA HAKKO KOGYO CO., LTD
Ohtemachi Bldg. Ohtemachi
1-chome Chiyoda-ku
Tokyo (JP)

(72) Inventor: Sakato, Kuniaki
1566-47, Nara-machi Midori-ku
Yokohama-shi Kanagawa-ken (JP)
Inventor: Tanaka, Hisao
3-6-6, Asahi-machi Machida-shi
Tokyo (JP)
Inventor: Motohashi, Ryoichi
165, Shimofujisawa
Iruma-shi Saitama-ken (JP)

(74) Representative: Casalonga, Axel, et al
BUREAU D.A. CASALONGA OFFICE JOSSE &
PETIT
Baaderstrasse 12-14
D-8000 München 5 (DE)

(56) References cited:
J. PHYS. E: SCI. INSTRUM., vol. 11, no. 6, June
1978, GREAT BRITAIN, J. C. S. RICHARDS et
al.: "Electronic measurement of bacterial
growth", pages 560-568

# 0 028 793

## Method for measuring metabolic activity of animal or plant tissues

Background of the invention

The present invention relates generally to a method for measuring the metabolic activity of animal or plant tissue and more specifically to a method for determining the number of active cells and metabolic activity or active parts of animal or plant tissue in a liquid containing such animal or plant tissue.

In the culturing an animal tissue, the measurement of cell propagation has heretofore been carried out by counting the number of cells or the number of cell nuclei cultured by an overlapping culturing method directly by a haemocytometer; by means of an electric cell number counter; or by a method which quantitatively and biochemically determines and evaluates components such as protein, nucleic acid, etc. The electric cell number counter provides a simple and rapid method, as compared with other methods requiring complicated test procedures, but counts not only living cells but also dead cells and suspended particles. In culturing a plant tissue, particularly in a suspension culture of cells, the bond between cells is so strong that the culture liquor comprises a suspension of aggregated cells and single cells. Thus, the propagation of cells is usually evaluated as fresh cell or dry cell weight. These weights are, however, not direct reflections of the number of active cells, but include dead cells and solid materials, such a disrupted cell walls and the like.

Culturing an animal and plant tissue is now being utilized for the production of food raw materials, medicaments and also for the production of physiologically active materials. It is a very important operation for the effective control of such production processes to measure the number of cells or metabolic activity originating from the animal or plant tissue in the culture liquor, and it is particularly desirable to make continuous measurements.

However, as described above, the conventional methods for measuring the number of animal or plant cells or metabolic activity are complicated in operation, and have such disadvantages as failure to make continous measurement, etc. and thus a novel, useful and simple method for measuring the metabolic activity of animal or plant tissue is in demand.

To this end, it has previously been found that, when an electrode was suspended in a culture liquor of microorganisms, a current or a potential originating from the microorganisms corresponded quite well to the metabolic activity of the microorganisms; such finding being disclosed in Japanese Patent Application No. 101,897/78 (Published Unexamined Japanese Patent Application No. 29940/80) and commonly owned German Offenlegungsschrift No. 29 33 994.

Summary of the invention

It has now been found by the present inventors that the metabolic activity of animal or plant tissue in a culture liquor can be determined by measuring a potential or a current originating from the animal or plant tissue through contact with an electrode. More specifically, it has now been found that a potential or a current measured by means of an electrode correlates well with the metabolic activity of the animal or plant tissue as determined by the heretofore known methods. Similarly, when the above-mentioned culture liquor is brought into contact with a potentiostatic system kept at a predetermined potential by two electrodes and a reference electrode (naturally the apparatus includes an apparatus producing a constant potential; this will be hereinafter applicable to any potentiostatic system) to measure the current originating from an animal or plant tissue, it was found that the measured current is in relation to metabolic activity.

In the present invention, the liquid containing an animal or plant tissue means a suspension of an animal or plant tissue, a culture liquor of an animal or plant tissue, and animal blood corpuscles, lymphocytes, etc.

The present invention thus provides a method for measuring metabolic activity of animal or plant tissue, characterized by contacting an electrode or a potentiostatic system with a culture liquor, and measuring a current or a potential generated through reaction of the animal or plant tissue with the electrode or the potentiostatic system.

A typical apparatus for carrying out the invention is an electrode having an anode and an internal electrolyte, a liquid junction for contact with an outside liquid, and an exposed cathode. Another apparatus is a potentiostatic system capable of maintaining an electrode comprising two electrodes and a reference electrode constant.

In any apparatus, a pair of electrodes or a potentiostatic system are used in combination, and a cathode of one electrode or potentiostatic system is covered with an animal or plant tissue-impermeable membrane, to apply to actual measurement. It was found that the difference in measured current value ($\Delta I$) or potential value ($\Delta E$) between two electrodes or potentiostatic systems corresponds well to the metabolic activity of the animal or plant cells.

For the electrode of the present invention, such metals as platinum, gold, silver, and the like are appropriate. As the reference electrode, silver–silver chloride electrode, calomel electrode and the like are appropriate.

2

**0 028 793**

Brief description of the drawings

Particular embodiments of the present invention have been chosen for purposes of illustration and are shown in the accompanying drawings wherein:

Fig. 1 is an elevation view, partially in cross-section, of one form of apparatus for carrying out the invention;

Fig. 2 is an elevation view, partially in cross-section, of another form of apparatus for carrying out the invention; and

Fig. 3 is a graph illustrating a particular relationship between metabolic activity of animal cells and current values obtained according to the invention.

Detailed description of the invention

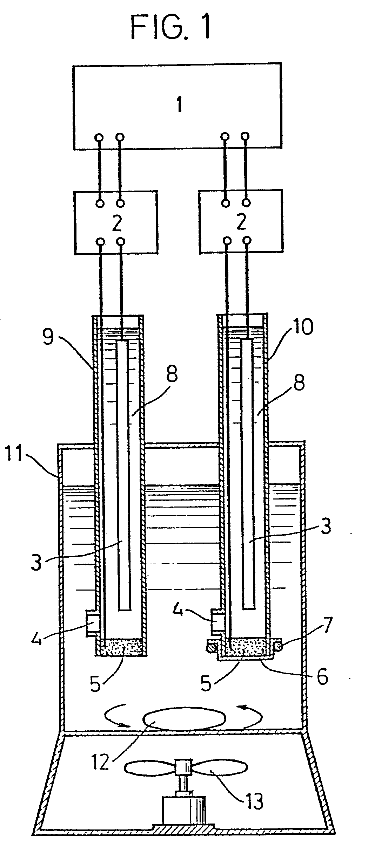

With reference to Fig. 1, the present invention is carried out using an apparatus having electrodes 9 and 10 each having an anode 3, an internal electrode 8 and a liquid junction 4 for contact with an outside liquid. One electrode 10, in which an exposed platinum electrode 5 is covered with an animal or plant tissue-impermeable membrane 6, is an electrode for measuring the electrode-active materials other than the animal or plant tissue.

The liquid junction 4 of electrodes 9 and 10 comprises a membrane having low electrical resistance, such as an ion exchange resin, ceramic, or the like. As the anode 3, metallic oxides, such as silver peroxide, silver chloride, silver dioxide, and the like or carbon or the like can be used. As the animal or plant cell-impermeable membrane 4 for covering the surface of the exposed platinum electrode 5, any animal or plant cell-impermeable membrane such as a cellulose membrane, a millipore membrane, etc. can be used, so long as it prevents the permeation of the animal or plant cells.

Reference numerals 2 and 1 indicate respectively an ammeter or a potentiometer and a recorder of standard design to which electrodes 9 and 10 are connected in standard fashion.

As illustrated, electrodes 9 and 10 are suspended in a housing 11 such as a culturing vessel equipped with, for example, a stirring bar 12 and magnetic stirrer 13, for carrying out measurements upon plant or animal tissue suspended in a liquid within housing 11.

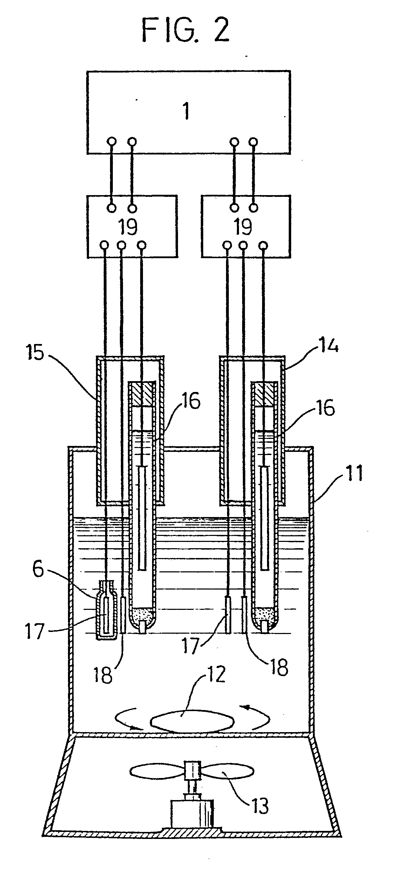

In Fig. 2 an alternate form of apparatus for measuring metabolic activity is illustrated and comprises a pair of potentiostatic systems (hereinafter referred to as "three-electrode method"). Each of the electrodes 14 and 15 of the individual potentiostatic systems has one reference electrode 16 for example, a calomel electrode and two electrodes 17 and 18. Housing 11 contains the culture liquor or tissue suspension into which the electrodes 14 and 15 are suspended. The exposed cathode 17 of one potentiostatic system 15 is covered with an animal or plant tissue-impermeable membrane 6, and is used for measuring electrode-active materials other than the animal or plant tissue. As the animal or plant tissue-impermeable membrane those membranes as mentioned above in reference to Fig. 1 may be used. Each pair of potentiostatic systems is connected to a potentiostat 19 which, in turn, is connected to a recorder 1.

As will be appreciated by those skilled in the art, the aforementioned two devices, can be simplified and modified, if required, and any apparatus structured as to have no adverse effect upon culturing animal or plant tissue cells and which can withstand the applied pressure, sterilization, etc. can be used.

According to the present invention, the metabolic activity of an animal or plant tissue in a culture liquor or suspension is measured by means of an apparatus as generally described above. That is, an animal or plant tissue and electrode-active materials, such as coenzyme, formic acid, hydrogen peroxide, hydrogen etc., in a culture liquor or suspension generate a current or a potential through contact with cathode 5 of electrode 9 or cathode 17 of electrode 14 of the potentiostatic system. On the other hand, only the electrode-active materials contact with the cathode 5 of electrode 10 or cathode 17 of electrode 15 of the potentiostatic system to generate a current or a potential.

The differences in currents or potentials measured as two electrodes 9 and 10 or electrodes 14 and 15 of two potentiostatic systems corresponds quite well to the number of cells or metabolic activity of the animal or plant tissue. Thus, the difference in current or potential between electrode 9 and electrode 10 or the difference in current or potential between electrodes 14 and 15 of the two potentiostatic systems is correlated to metabolic activity of an animal or plant tissue. Therefore, the metabolic activity of the animal or plant tissue can be reliably measured from these values.

While the principal and exact mechanism of the phenomena which the present invention utilizes is not fully understood, there is nevertheless a reliable correlation between the current or potential originating from animal or plant tissue and metabolic activity which permits determination of such metabolic activity.

Certain specific embodiments of the present invention are illustrated in the following representative examples.

Example 1

In this example, human origin cancer cells, i.e. KB cells, were inoculated into Eagle MEM medium (pH 7.2), placed in a square-type culturing bottle and subjected to stationary culture in a carbon dioxide

3

incubator at 37°C, and sampled every other day. The number of cells were counted with a haemocytometer.

Simultaneously, an apparatus for measuring metabolic activity comprising two electrodes 9 and 10, shown in Fig. 1, with disk-type platinum electrodes (1 cm in diameter) as cathode, silver peroxide electrodes 8 having a potential of 276 mV against saturated calomel electrode (5 mm in outer diameter and 14 cm in length), as anode, anion exchange membrane (Selemion type AMV, made by Asahi Glass K.K.) as liquid junction 4, 1 m phosphate buffer solution (pH 7.0) as an internal electrolyte, and dialysis cellulose membrane as the covering membrane for the surface of cathode 10 of one of the electrodes 10 was suspended in the sampled liquid. The change in difference in current values ($\Delta I$) obtained between the two electrodes with time is given in Table 1 below.

TABLE 1

| Culturing day | 2 | 4 | 6 | 8 |
|---|---|---|---|---|
| Number of cells ($\times 10^4$/ml) | 10.8 | 35.7 | 60.1 | 86.5 |
| Current value $\Delta I$ (nA/cm$^2$) | 0.02 | 0.06 | 0.09 | 0.11 |

The results show a good correlation between the current value and the number of cells (living residue: 93.4%).

Example 2

In this example, cancer cells L-1210 were inoculated into RPMI-1640 liquid medium (pH 7.2), placed in a square type culturing bottle and cultured at 37°C for one week in a carbon dioxide incubator. The cell-containing liquid was diluted with physiological saline water, and the number of cells were counted by means of an electric cell counter.

At the same time, the current of the diluted, cell-containing liquid was measured according to the three-electrode method with a pair of potentiostatic systems comprising platinum electrodes 17, platinum electrodes 18 and silver chloride electrodes 16 (reference electrodes), shown in Fig. 2 (the cathode 17 of the electrode 15 of one potentiostatic systems was covered with a dialysis cellulose membrane), and the relation between the number of cells and the difference in current values ($\Delta I$) obtained between the pair of potentiostatic systems is shown in Table 2 below.

TABLE 2

| Number of cells ( /ml) | $10^3$ | $10^4$ | $10^5$ | $10^6$ |
|---|---|---|---|---|
| Current value $\Delta I$ (nA) | 213 | 116 | 31 | 10 |

The results in Table 2 are examples measured under an applied voltage of −500 mV, and a good correlation was established between the number of cells and the difference in current values ($\Delta I$) obtained.

Example 3

In this example, 0.2 ml of DDY-strain mouse blood was sampled, and admixed with 10 ml of physiologically saline water. The supernatant was admixed with a drop of Saponin solution to perform hemolysis. The number of leukocytes was counted by means of an electric cell counter. On the other hand, the current of the leukocyte-containing solution was measured according to the three-electrode method, using the same apparatus as used in Example 2. The relationship between the number of leukocytes and the difference in current values ($\Delta I$) are shown in Table 3 below.

TABLE 3

| Number of leukocytes ( /mm$^3$) | 600 | 3400 | 17500 | 84500 |
|---|---|---|---|---|
| Current value $\Delta I$ (nA) | 160 | 190 | 280 | 712 |

The results in Table 3 were obtained under an applied voltage of −550 mV, and correlations were established between the number of leukocytes and the difference in current values ($\Delta I$) obtained.

Example 4

In this example, callus of Vinca rosea was inoculated into 100 ml of Murashige-Skoog liquid medium containing 0.5 ppm of kinetin and 0.1 ppm of 2,4-dichlorophenoxyacetic acid, placed in a 300 ml Erlenmeyer flask and cultured at 30°C in a shaking cultivator at 100 rmp. The current value of the culture liquor was measured by means of the same apparatus with two electrodes as used in Example 1, and compared with the fresh cell weight (FCW) determined by standard procedure. The results are shown in Table 4 below.

TABLE 4

| Culturing day | 1 | 6 | 10 | 15 | 20 |
|---|---|---|---|---|---|
| FCW (mg/ml) | 10.2 | 50 | 176 | 321 | 334 |
| Current value $\Delta I$ (nA) | −9.5 | −9.2 | −6.7 | −3.0 | −3.1 |

4

A good correlation was observed between FCW and the difference in current values (ΔI). Similarly, the results obtained according to the three-electrode method by means of the same apparatus as used in Example 2 are shown in Table 5 below.

TABLE 5

| Culturing day | 1 | 6 | 10 | 15 | 20 |
|---|---|---|---|---|---|
| FCW (mg/ml) | 10.2 | 50 | 176 | 321 | 334 |
| Current value ΔI (nA) | 10 | 15 | 27 | 31 | 32 |

Table 5 shows the results obtained under an applied voltage of −200 mV, and a good correlation was observed between FCW and the difference in current values (ΔI) obtained, as in Table 4.

Example 5

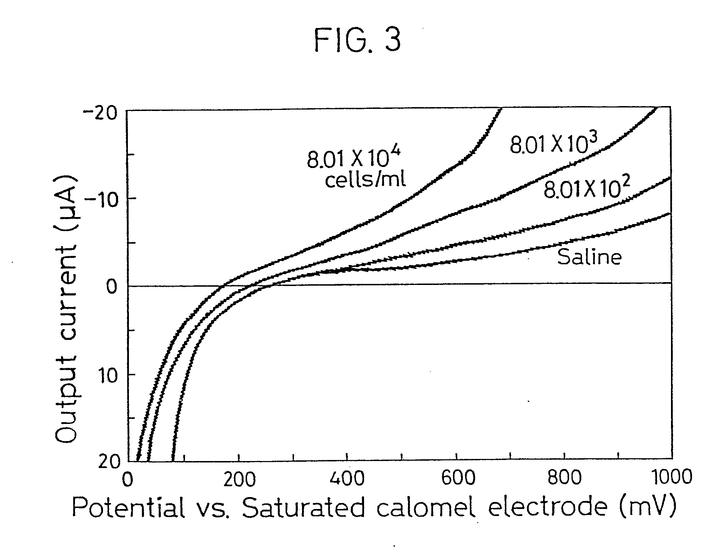

In this example, Namalwa cells were incubated in a spinner containing RPMI-1640 medium (pH 7.2) comprising 20% foetal calf serum and 100 units of Penicillin and Streptomicin at 36°C for 24 hours. The number of viable cells were counted with a haemocytometer chamber after straining with 0.2% Erythrocin. Fig. 3 shows the polarogram of various Namalwa cell suspensions which were diluted with normal saline (0.85% NaCl). As is apparent from Fig. 3, the output currents from a potentiostat in the range of more than +400 mV depend on the number of viable cells.

**Claims**

1. A method for determining the metabolic activity of animal or plant tissue in a liquid medium comprising electrode-active materials which comprises measuring the difference between the current value of potential value of said animal or plant tissue and electrode-active materials and the current value or potential value of the electrode-active materials only.

2. A method according to claim 1 wherein said measurement is carried out by suspending two electrodes in said liquid medium, one of said electrodes being covered with a membrane impermeable to said animal or plant tissue and comparing the current value or potential value of said two electrodes.

3. A method according to claim 1 wherein said measurement is carried out by suspending two potentiostatic systems each comprising two exposed electrodes and a reference electrode in said liquid medium, one of said potentiostatic systems having one of its exposed electrodes covered with a membrane impermeable to said animal or plant tissue and comparing the current or potential of said two potentiostatic systems.

4. Apparatus for determining the metabolic activity of animal or plant tissue in a liquid medium which comprises two electrodes each of said electrodes comprising an anode and an internal electrolyte, a liquid junction with said outside liquid medium and an exposed cathode, one of said cathodes being covered with an animal or plant tissue-impermeable membrane, each electrode being connected to an ammeter or potentiometer and to a recorder.

5. Apparatus for determining the metabolic activity of animal or plant tissue in a liquid medium which comprises two potentiostatic systems each of them comprising one reference electrode and two exposed electrodes, the exposed cathode of one of said systems being covered with an animal or plant tissue-impermeable membrane, each potentiostatic system being connected to a potentiostat which, in turn, is connected to a recorder.

**Patentansprüche**

1. Verfahren zur Bestimmung der Stoffwechseltätigkeit tierischen oder pflanzlichen Gewebes in einem flüssigen, elektrodenaktive Materialien enthaltenden Medium, bestehend aud dem Messen des Unterschieds zwischen dem Stromwert bzw. dem Potentialwert des gennanten tierischen bzw. pflanzlichen Gewebes und der elektrodenaktiven Materialien und dem Stromwert bzw. Potentialwert der elektrodenaktiven Materialien allein.

2. Verfahren gemäss Anspruch 1, wobei die gennannte Messung ausgeführt wird, indem man zwei Elektroden in das genannte flüssige Medium hängt, von denen eine mit einer für das genannte tierische bzw. pflanzliche Gewebe undurchlässigen Membran überzogen ist, und den Stromwert bzw. Potentialwert dieser beiden Elektroden vergleicht.

3. Verfahren gemäss Anspruch 1, wobei die genannte Messung ausgeführt wird, indem man zwei potentiostatische Systeme, von denen jedes zwei freiliegende Elektroden und eine Bezugselektrode enthält, in das genannte flüssige Medium hängt, wobei bei einem dieser potentiostatischen Systeme eine ihrer freiliegenden Elektroden mit einer für das genannte tierische bzw. pflanzliche Gewebe undurchlässigen Membran überzogen ist, und den Strom bzw. das Potential der genannten zwei potentiostatischen Systeme vergleicht.

4. Apparat zur Bestimmung der Stoffwechseltätigkeit tierischen oder pflanzlichen Gewebes in einem flüssigen Medium, welches zwei Elektroden enthält, von denen jede eine Anode und einen internen Elektrolyten, eine Flüssigkeitskontaktstelle mit dem genannten äusseren flüssigen Medium und

5

**0 028 793**

eine freiliegende Kathode umfasst, wobei eine der genannten Kathoden mit einer für das tierische bzw. pflanzliche Gewebe undurchlässigen Membran überzogen ist, und jede Elektrode an einen Amperemeter bzw. einen Potentiometer und einen Schreiber angeschlossen ist.

5. Apparat zur Bestimmung der Stoffwechseltätigkeit tierischen oder pflanzlichen Gewebes in einem flüssigen Medium, das zwei potentiostatische Systeme enthält, von denen jedes eine Bezugselektrode und zwei freiliegende Elektroden umfasst, wobei die freiliegende Kathode eines dieser Systeme mit einer für das tierische bzw. pflanzliche Gewebe undurchlässigen Membran überzogen ist, und jedes potentiostatische System mit einem Potentiostat verbunden ist, der seinerseits an einen Schreiber angeschlossen ist.

## Revendications

1. Procédé de détermination de l'activité métabolique d'un tissu animal ou végétal dans un milieu liquide comprenant des matières actives d'électrode, caractérisé en ce qu'il consiste à mesurer la différence entre la valeur du courant ou la valeur du potentiel du tissu animal ou végétal et des matières actives d'électrode, et la valeur du courant ou la valeur du potentiel des matières actives d'électrode seulement.

2. Procédé selon la revendication 1, caractérisé en ce que la mesure est effectuée en suspendant deux électrodes dans le milieu liquide, l'une des électrodes étant recouverte d'une membrane imperméable au tissu animal ou végétal, et en comparant la valeur du courant ou la valeur du potentiel de ces deux électrodes.

3. Procédé selon la revendication 1, caractérisé en ce que la mesure est réalisée en suspendant dans le milieu liquide deux systèmes potentiostatiques, chacun comprenant deux électrodes exposées et une électrode de référence, l'un de ces systèmes potentiostatiques ayant l'une de ses électrodes exposées recouverte d'une membrane imperméable au tissu animal ou végétal, et en comparant le courant ou le potentiel des deux systèmes potentiostatiques.

4. Appareil pour déterminer l'activité métabolique d'un tissu animal ou végétal dans un milieu liquide, caractérisé en ce qu'il comprend deux électrodes, chacune des électrodes comprenant une anode et un électrolyte interne, un raccordement liquide avec le milieu liquide extérieur et une cathode exposée, l'une des cathodes étant recouverte d'une membrane imperméable au tissu animal ou végétal, chaque électrode étant raccordée à un ampèremètre ou à un potentiomètre et à un enregistreur.

5. Appareil pour déterminer l'activité métabolique d'un tissu animal ou végétal dans un milieu liquide, caractérisé en ce qu'il comprend deux systèmes potentiostatiques, chacun d'eux comprenant une électrode de référence et deux électrodes exposées, la cathode exposée de l'un des systèmes étant recouverte d'une membrane imperméable au tissu animal ou végétal, chaque système potentiostatique étant raccordé à un potentiostat lequel à son tour, est raccordé à un enregistreur.

# FIG. 1

# FIG. 2

# FIG. 3